(19) Europäisches Patentamt
European Patent Office
Office européen des brevets

(11) Veröffentlichungsnummer : **0 066 885**
**B1**

(12) # EUROPÄISCHE PATENTSCHRIFT

(45) Veröffentlichungstag der Patentschrift : **09.10.85**

(51) Int. Cl.⁴ : **C 07 C 89/02**, C 07 C 91/04

(21) Anmeldenummer : **82105029.1**

(22) Anmeldetag : **08.06.82**

(54) **Verfahren zur Herstellung von N-/2-Aminobenzyl/-1-Phenyl-2-Methyl-Aminoäthanol.**

(30) Priorität : **10.06.81 BG 52398/81**

(43) Veröffentlichungstag der Anmeldung : **15.12.82 Patentblatt 82/50**

(45) Bekanntmachung des Hinweises auf die Patenterteilung : **09.10.85 Patentblatt 85/41**

(84) Benannte Vertragsstaaten : **BE CH DE FR GB IT LI NL**

(56) Entgegenhaltungen :
**DE-A- 1 670 694**
**FR-M- 7 206**

(73) Patentinhaber : **DSO "PHARMACHIM"**
**16, Iliensko Chaussee**
**Sofia (BG)**

(72) Erfinder : **Sabunova, Orchideja Bontscheva**
**Komplex Mladost-2, Block 215-5**
**Sofia (BG)**
Erfinder : **Ivanov, Tschavdar Borissov**
**Komplex Mladost-1, Block 46-4**
**Sofia (BG)**
Erfinder : **Mondeschka, Donka Minkova**
**Rakovski-Strasse 125**
**Sofia (BG)**
Erfinder : **Georgiev, Alexander Hristov**
**Botevgradsko Chussée, Block 14-1**
**Sofia (BG)**
Erfinder : **Dimov, Neno Pentschev**
**Komplex Mladost-1, Block 66-A**
**Sofia (BG)**
Erfinder : **Agapova, Nelly Nedeltscheva**
**Alabin-Strasse 50-2**
**Sofia (BG)**
Erfinder : **Pavlova, Susana Georgieva**
**6-ti Septemvri-Strasse**
**Sofia (BG)**

(74) Vertreter : **von Füner, Alexander, Dr. et al**
**Patentanwälte v. Füner, Ebbinghaus, Finck Mariahilfplatz 2 & 3**
**D-8000 München 90 (DE)**

**EP 0 066 885 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von N-(2-Aminobenzyl)-1-phenyl-2-methylaminoäthanol, ein Zwischenprodukt in der Synthese des Präparats « Alival » (Nomiphenzin), das für medizinische Zwecke als ein antidepressantes Mittel verwendet wird.

Es ist bekannt, daß N-(2-Aminobenzyl)-1-phenyl-2-methylaminoäthanol, ein Zwischenprodukt für die Herstellung des Präparats « Nomiphenzin », in drei Stufen erhalten wird : Alkylieren des 2-Nitrobenzylmethylamins mit α-Bromazetophenon, wobei als Ergebnis N-(2-Nitrobenzyl)-methylaminoazetophenon hergestellt wird ; letzteres wird katalytisch mit Wasserstoff zu N-(2-Aminobenzyl)-methylaminoazetophenon reduziert, das nach Reaktion mit Natriumborhydrid in den obengenannten Aminoalkohol umgewandelt wird, wie dem Schema 1 zu entnehmen ist.

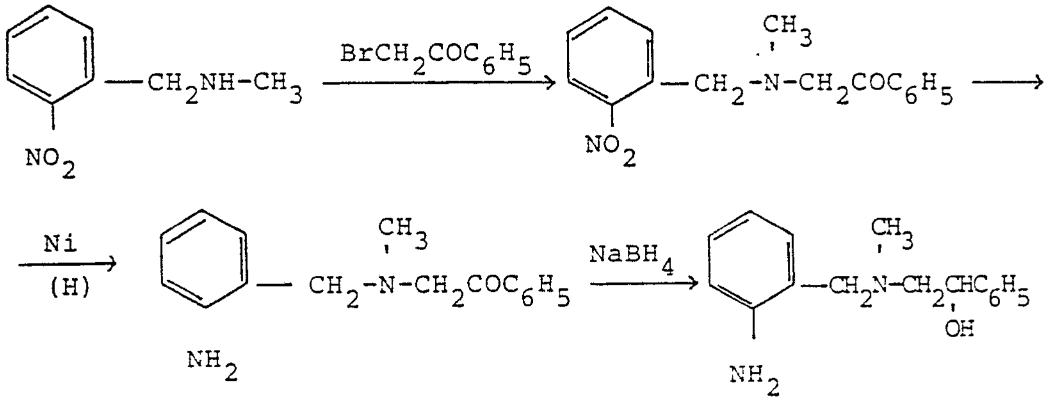


Als Nachteile dieser Reaktion sind die mehrstufige Herstellung des Aminokarbinols und die Verwendung des starken augenreizenden Mittels Bromazetophenon, das wesentlich die industrielle Anwendung hemmt, zu nennen.

In der FR-M-7.206 wird ein Verfahren zur Herstellung von 1,2-Aminoalkoholen beschrieben, das auf der Umsetzung von p-Nitrostyroloxid mit aliphatischen Aminen, wie Allylamin, Diisopropylamin, Morpholin und Pyridin, in Äthylalkohol beim Sieden des Reaktionsgemisches beruht. Diese Reaktionsbedingungen für die Umsetzung zwischen Styroloxid und Benzylamin sind in J. Chem. Soc. 6065-6, 1963 und J. Chem. Soc. 2569-79, 1965 beschrieben, wo bewiesen ist, daß die Reaktion unter Bildung von 2 Isomeren, nämlich normalem A-Isomer und dem anormalen B-Isomer verläuft. Es ist zuzugeben, daß nach der Lehre der FR-PS angenommen werden könnte, daß dort ebenfalls 2 Isomere erhalten werden. In der Beschreibung ist darüber allerdings nichts gesagt und es ist auch keine Literaturstelle genannt, die dieses zuläßt. Aus diesem Grund kann nicht davon ausgegangen werden, daß der allgemeinen Lehre der FR-M-7.206 entnommen werden kann, die Reaktion verlaufe selektiv und führe ausschließlich zum A-Isomer. In der FR-PS sind nämlich keine Daten wie Infrarot oder kernmagnetische Resonanzspektren oder auch Dünnschichtchromatogramme angeführt, die die Selektivität der bekannten Reaktion beweisen. Es ist bekannt, daß bei chemischen Reaktionen zwar Spekulationen angestellt werden, was geschehen könnte, wenn. Ohne entsprechende chemisch-physikalische Daten ist ein derart angenommener Reaktionsverlauf jedoch nie bewiesen. In dem zu untersuchenden Fall der FR-PS sind nur Schmelzpunkte angegeben, was nicht ausreicht, von der gesicherten Erkenntnis auszugehen, daß die Reaktion selektiv ist. Es ist bekannt, daß die 1,2-Aminoalkoholisomere sich in ihren Schmelzpunkten kaum unterscheiden und somit keinen Schluß zulassen, ob und welche Isomere vorliegen.

Die diesseitige Ansicht, daß die bekannte Reaktion nicht selektiv verläuft, wird außerdem noch durch die Tatsache gestützt, daß die Ausbeute der erhaltenen 1,2-Aminoalkohole ziemlich niedrig ist, und zwar 60 bis 65 % der Theorie. Dies spricht eindeutig für die Bildung von 2 Isomeren. Im übrigen muß man auch unterscheiden zwischen der Bildung von optisch aktiven Isomeren und Positionsisomeren.

Weiterhin ist es nicht belanglos, wovon man bei einer Reaktion ausgeht, also vom Styroloxid und dieses mit 2-Nitrobenzylmethylamin umsetzt oder vom substituierten Styroloxid und dieses gemäß dem Stand der Technik mit aliphatischen Aminen reagieren läßt. Es gibt kein Standardverfahren zur Umsetzung von Aminen mit Styroloxid. Es besteht ein Unterschied zwischen aliphatischen und aromatischen Aminen etc. Man kann also von vornherein nicht vorhersehen, wie je nach Einsatz von Ausgangsprodukten eine Reaktion verlaufen wird und insbesondere ob sie eine hohe Selektivität gewährleistet.

In den oben zitierten Literaturstellen wird ebenfalls keine allgemeine Lehre gegeben, sondern es werden konkrete Fälle abgehandelt. So wird beispielsweise in « Journal Chemical society », 2569-79, 1965, der Einfluß verschiedener Reaktionsparameter auf die Umsetzung von Benzylamin und Styrol untersucht. In « J. Chem. Soc. », 4277C, 83, werden Untersuchungen über die Umsetzung zwischen substituierten Benzylaminen und Styroloxid beschrieben. Dies allein zeigt, daß der Ablauf einer Reaktion

zwischen zwei bestimmten Ausgangsprodukten in dem zu untersuchenden Fall nicht vorhergesehen werden kann ; insbesondere was Selektivität und Ausbeute angeht und nur durch Versuche ermittelt werden kann. Es gibt keine allgemeingültigen Abhängigkeiten der Parameter für die Reaktion.

Bei Anwendung der aus der Literatur bekannten Reaktionsbedingungen wie Reaktionsmedium Äthanol und Siedepunkt des Reaktionsgemisches, erhält man eine niedrige Selektivität, wobei das Verhältnis der Isomeren A : B = 70 : 30 beträgt.

Nimmt man also die für die Umsetzung zwischen Benzylamin und Styroloxid vorgeschlagenen Lösungsmittel wie Äthanol, Methanol, Propanol etc (Journal Chemical Society 2569-79, 1965), so bekommt man als größtes Verhältnis der beiden Isomere 70 : 30 und damit keine hohe Selektivität und eine wirtschaftliche Begrenzung der Verfahrens. Nimmt man andererseits Lösungsmittel vom Typ Acetonitril, Nitrobenzol, Tetrahydrothiophen-1,1-dioxid, so kann man eine höhere Selektivität erreichen, aber dies auf Kosten einer wesentlich niedrigen Reaktionsgeschwindigkeit, so daß man wiederum nicht zu einem industriell durchführbaren Verfarhren gelangt.

Demgegenüber erhält man erfindungsgemäß eine hohe Selektivität bei einem Verhältnis der Isomeren A : B = 90 : 10 durch Anwendung von Dimethylformamid. Die Reaktionsgeschwindigkeit ist hoch, was das Verfahren wirtschaftlich äußerst interessant und industriell anwendbar macht. Die hohe Selektivität des erfindungsgemäßen Verfahrens sichert eine hohe Ausbeute und Reinheit des Endprodukts, und zwar von 8-Amino-4-phenyl-2-methyl-1,2,3,4-tetrahydroisochinolinmaleat, was bisher nach dem bekannten Verfahren nicht erreicht werden konnte. Im übrigen zeigen die Beispiele — Beispiel 1 mit Äthanol 70 % und Beispiel 2 mit Dimethyl — formamid 85 % — die behauptete Steigerung eindeutig.

Der Erfindung liegt also die Aufgabe zugrunde, ein neues hochwirksames und industriell anwendbares Verfahren zur Herstellung von N-(2-Aminobenzyl)-1-phenyl-2-methylaminoäthanol zu schaffen.

Erfindungsgemäß wird diese Aufgabe dadurch gelöst, daß man 2-Nitrobenzylmethylamin mit Styroloxid in einem Medium von Dimethylformamid umsetzt und das so erhaltene N-(2-Nitrobenzyl)-1-phenyl-2-methylaminoäthanol einer direkten katalytischen Hydrierung unterwirft, wie aus dem folgenden Schema zu sehen ist :

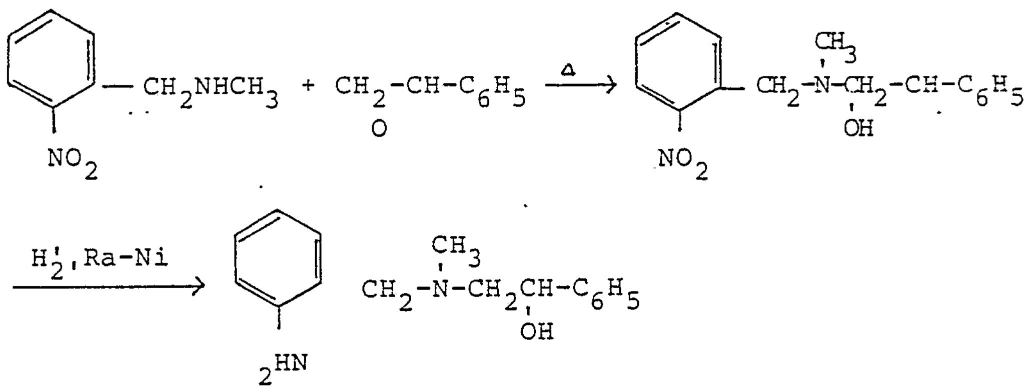


Die Reaktion zwischen 2-Nitrobenzylmethylamin und Styroloxid erfolgt zweckmäßig in einem Temperaturintervall von 60 bis 150 °C. Die Zeitdauer der Reaktion liegt bei 6 bis 20 Stunden. Die molaren Verhältnise variieren von 1 : 1 bis 1 : 2 zugunsten des Styroloxids.

Die Ausbeute an Endprodukt beträgt bei Verwendung von Dimethylformamid 84 %, die Reinheit beträgt ebenfalls 84 %.

Diese Lösungsmittel sind bei den vom Stand der Technik bekannten Verfahren zur Herstellung von 1,2-Aminoalkoholen als solche nicht beschrieben.

Wesentlich niedrigere Ergebnisse werden bei der Durchführung der Reaktion in den vom Stand der Technik bekannten Lösungsmitteln, wie zum Beispiel Äthanol, erhalten. In diesem Fall beträgt die Ausbeute an N-(2-Aminobenzyl)-1-phenyl-2-methylaminoäthanol 72 % und der prozentuelle Gehalt 70 %.

Das katalytische Hydrieren des sich zwischenbildenden N-(2-Nitrobenzyl)-1-phenyl-2-methylaminoäthanol wird zweckmäßig in einem Medium von Äthanol bei Luftdruck und Zimmertemperatur durchgeführt. Somit wird das Dimethylformamid abdestilliert, wonach das Hydrieren in Äthanol erfolgt.

Die Reinheit des N-(2-Aminobenzyl)-1-phenyl-2-methylaminoäthanols wurde mit Hilfe einer hochwirksamen Flüssigchromatographie und kernmagnetischer Resonanzspektroskopie bestimmt. Das erfindungsgemäße Verfahren weit im Vergleich zu den vom Stand der Technik bekannten Verfahren zur Herstellung der 1,2-Aminoalkohole folgende Vorteile auf.

1. Das Verfahren gewährleistet eine hohe Ausbeute und Reinheit des N-(2-Aminobenzyl)-1-phenyl-2-methylaminoäthanol durch den Einsatz von Dimethylformamid in der ersten Reaktionsstufe. Solche Ergebnisse können nicht bei Anwendung von Lösungsmitteln, wie z. B. Äthanol allein erreicht werden, die bei den bis z. Z. bekannten Verfahren zur Herstellung von 1,2-Aminoalkoholen benutzt werden (FR-PS 7206 M, J. Chem. Soc. 6065-7, 1963). Dies wird durch die nachstehend angegebenen Vergleichsergebnisse bewiesen.

2. Im Vergleich zu dem Verfahren zur Herstellung von N-(2-Aminobenzyl)-1-phenyl-2-methylaminoäthanol (GB-PS-1 164 192) zeichnet sich das erfindungsgemäß Verfahren dadurch aus, daß die Zahl der Reaktionsstufen vermindert wird, das kostspielige Reagenz α-Bromazetrophenon entfällt und daß der technologische Vorgang wesentlich vereinfacht wird.

Vergleichsbeispiel 1

26 g (0,16 Mol) von 2-Nitrobenzylmethylamin und 22,8 g (0,19 Mol) Styroloxid werden in 250 ml Äthylalkohol gelöst und das Reaktionsgemisch wird bei 80 °C während 10 Stunden erwärmt. Nach Beendigung der Reaktion wird das Gemisch bis zur Zimmertemperatur abgekühlt, dann werden 2,6 g Raney-Nickel zugefügt und mit Wasserstoff bei Atmosphärendruck und Zimmertemperatur bis zur Aufnahme der theoretischen Stoffmenge hydriert. Der Katalysator wird abfiltriert, man wäscht mit Äthanol und konzentriert das Filtrat bis zum Trocknen unter Vakuum. Es werden 41,3 g N-(2-Aminobenzyl)-1-phenyl-2-methylaminoäthanol in Form eines viskosen Öls mit einer Reinheit von 70 % erhalten. Die Ausbeute an 100 % Aminoalkohol beträgt 28,91 g oder 72 % der theoretischen Menge. Das kernmagnetische Resonanz-Spektrum ist in einer 10 %-Lösung von $\alpha_1$-$CHCl_3$, die 1 % HMSO als Internstandard enthält, aufgenommen, mit Hilfe eines Spektrometers Model INM-PS-100, das auf einer Frequenz von 100 MHz arbeitet. Die chemischen Versetzungen der Protonen sind in ppm (Teil pro Million) angegeben. In dem Spektrum wurden folgende Linien beobachtet: N—$CH_3$-2,24 ppm/c, 3H/, —$CH_2$CH-2,30-2,76 ppm und 4,60-4,76 ppm/m, 3H, System ABX, $J_{AX}$ = 4 Hz und $J_{BX}$ = 9,5 Hz, Ar—$CH_2$—N-3,37 ppm und 3,56 ppm/m, 2H, System AB, J = 15 Hz, OH und $NH_2$-3,80 ppm/m (erweitertes Singlet), O—$C_6H_4$-6,48-7,10 ppm,/4H, System ABSD, $C_6H_5$-7,22 ppm (c, 5H).

Beispiel 2

27,7 g (0,17 Mol) von 2-Nitrobenzylmethylamin und 22,85 g (0,19 Mol) Styroloxid werden in 150 ml Dimethylformamid gelöst und das Reaktionsgemisch bei 100 °C während 20 Stunden erwärmt. Nach Beendigung der Reaktion wird das Dimethylformamid unter Vakuum destilliert und zu dem bleibenden Rückstand werden 170 ml Äthylalkohol und 3 g Raney-Nickel zugegeben. Man hydriert mit Wasserstoff bei Atmosphärendruck und Zimmertemperatur bis zur Aufnahme der theoretischen Menge. Der Katalysator wird abfiltriert, man wäscht mit Äthanol und konzentriert das Filtrat bis zum Trocknen unter Vakuum. Es werden 42,7 g N-(2-Aminobenzyl)-1-phenyl-2-methylaminoäthanol in Form eines viskosen Öls mit Reinheit 84 % erhalten. Die Ausbeute an 100 % Aminoalkohol beträgt 36 g oder 84 % der theoretischen Menge.

Vergleichstabelle über den Einfluß der organischen Lösungsmitel auf die Ausbeute und die Reinheit des N-(2-Aminobenzyl)-1-phenyl-2-methylaminoäthanol

| Beispiel | organische Lösungsmittel | prozentueller Gehalt | Ausbeute in % |
|---|---|---|---|
| 1 | Äthanol | 70 | 72 |
| 2 | Dimethylformamid | 84 | 84 |

**Patentanspruch**

Verfahren zur Herstellung von N-(2-Aminobenzyl)-1-phenyl-2-methylaminoäthanol, dadurch gekennzeichnet, daß man 2-Nitrobenzylmethylamin mit Styroloxid in einem Medium von Dimethylformamid umsetzt und das so erhaltene N-(2-Nitrobenzyl)-1-phenyl-2-methylaminoäthanol einer direkten katalytischen Hydrierung in Äthanol unterwirft.

**Claim**

Process for the production of N-(2-aminobenzyl)-1-phenyl-2-methylaminoethanol, characterised in that 2-nitrobenzylmethylamine is reacted with styrene oxide in a dimethylformamide medium and the N-(2-nitrobenzyl)-1-phenyl-2-methylaminoethanol so obtained is subjected to a direct catalytic hydrogenation in ethanol.

**Revendication**

Procédé de préparation du N-(2-aminobenzyl)-1-phényl-2-méthylaminoéthanol, caractérisé en ce que l'on fait réagir de la 2-nitrobenzylméthylamine avec de l'oxyde de styrolène dans un milieu de diméthylformamide et l'on soumet le N-(2-nitrobenzyl)-1-phényl-2-méthylaminoéthanol ainsi obtenu à une hydrogénation catalytique directe dans l'éthanol.